# EUROPEAN PATENT APPLICATION

(11) **EP 4 772 140 A1**
(43) Date of publication of application: **08.07.2026**
(21) Application number: 25227046.7
(22) Date of filing: 23.12.2025
(51) Int. Cl.: A61C 19/06, A61N 5/06

(54) **INTRAORAL LIGHT THERAPY DEVICE**

(30) Priority: 06.01.2025 JP 2025002017; 07.08.2025 JP 2025132600
(71) Applicant: Japan PBM Healing Kabushiki Kaisha, Tokyo 108-0023 (JP)
(72) Inventor: KWONG HING, Alan, 75004 Paris (FR)
(74) Representative: Gislon, Gabriele

(57) **Abstract**

An object of the present invention is to provide an intraoral light therapy device that not only accurately irradiates light to gingival margin and free gingiva throughout a user's entire dentition, but also provides excellent wearing comfort when the user fits the mouthpiece deep inside the oral cavity.

An intraoral light therapy device comprises a mouthpiece (1), and a light source (2) disposed in the mouthpiece (1) and provided with a plurality of light emitters (20), the mouthpiece (1) comprising a bite tray (3) formed in a U-shape according to a wearer's dentition and bitten by the dentition of the wearer's upper and lower jaws, a front flange (4) facing outer surfaces of the wearer's upper and lower gums (9), and a rear flange (5) facing inner surfaces of the wearer's upper and lower gums (9). Each outer edge end portion (30a) of rear end portions (30, 30) of the bite tray (3) is formed flush with a tip portion of a rear end portion (4b) of the front flange (4) to which the outer edge end portion (30a) is connected, and both rear end portions (5b, 5b) of the rear flange (5) are located anterior to the outer edge end portions (30a, 30a) of both the rear end portions (30, 30) of the bite tray (3).

## Description

### FIELD OF THE INVENTION

The present invention relates to an intraoral light therapy device, particularly to an intraoral light therapy device that not only accurately irradiates light to gingival margin and free gingiva throughout a user's entire dentition, but also provides excellent wearing comfort when the user fits the mouthpiece deep inside the oral cavity.

### BACKGROUND OF THE INVENTION

Various dental light irradiation devices have been proposed to promote sterilization and bacteria elimination in an oral cavity by light irradiation and to restore healthy conditions for diseases such as periodontal disease. For example, as a dental light irradiation device in which a lot of light-emitting diodes are arranged along dentition to irradiate light to necessary areas efficiently, JP 2000-70292 A proposes a dental light irradiation device including a mouthpiece that has a concave portion into which dentition is inserted to interpose the dentition from the front and rear, a plurality of light-emitting diodes that is provided on the inside the concave portion of the mouthpiece such that light-emitting surfaces of them are arranged along the dentition, and a switch turning the light-emitting diodes ON/OFF. The device of JP 2000-70292 A has a plurality of light-emitting diodes emitting light of a wavelength of 470 nm or less with strong bactericidal action, and another plurality of light-emitting diodes emitting light in the infrared range to give a thermal stimulus to gums and improve blood circulation in the gums.

JP 2022-553650 A proposes an oral treatment device comprising an intraoral mouthpiece, comprising:
- a body made of a heat-conducting material, the body further having an upper surface and an opposite lower surface, said surfaces comprising an essentially planar portion between lingual edges and buccal or labial edges, respectively, and configured to be placed against teeth surfaces, and
- a plurality of light sources attached to the body and adapted to deliver light to teeth surfaces, wherein
- said edges comprise surfaces, at least a part of which are planar and tilted so as to form in cross-section of the body an angle of 5 to 85° with respect to the planar portions of the upper and lower surfaces,
- at least a part of the light sources are positioned on at least one of the tilted buccal, labial or lingual edges of the body, and
- the body and the light sources are embedded in an encasing formed by a transparent polymeric material.

The device of JP 2022-553650 A is configured to irradiate light of a wavelength of 405 nm and light of a wavelength of 780 to 820 nm. Moreover, JP 2022-553650 A describes that a photosensitizer may also be used when irradiating with light.

Nevertheless, such an intraoral light therapy device of mouthpiece type is required not only to accurately irradiate a gingival margin and a free gingiva over an entire dentition from front teeth to molars, but also to improve a wearing comfort, for example, reducing discomfort and vomiting reflex when the mouthpiece is fit into a user's oral cavity. Particularly, for treating periodontal disease in molars and wisdom teeth, a shape that accounts for the wearing comfort when the mouthpiece is fit to the deep interior of the oral cavity is required. However, neither of the devices of Patent Documents 1, 2 provides a good wearing comfort for the mouthpiece thereof, making it difficult to sufficiently reduce discomfort and vomiting reflex.

### SUMMARY OF THE INVENTION

### PROBLEMS TO BE SOLVED BY THE INVENTION

In view of the above disadvantages, an object of the present invention is to provide an intraoral light therapy device that not only accurately irradiates light to gingival margin and free gingiva throughout a user's entire dentition, but also provides excellent wearing comfort when the user fits the mouthpiece deep inside the oral cavity.

### MEANS FOR SOLVING THE PROBLEMS

As a result of intense research in view of the above object, the inventor has found that when each outer edge end portion of rear end portions of a bite tray is formed flush with a tip portion of a rear end portion of a front flange to which the outer edge end portion is connected, and both rear end portions of a rear flange are located anterior to the outer edge end portions of both the rear end portions of the bite tray, it is possible to obtain an intraoral light therapy device that not only accurately irradiates light to gingival margin and free gingiva throughout a user's entire dentition, but also provides excellent wearing comfort when the user fits the mouthpiece deep inside the oral cavity, too.

Thus, the intraoral light therapy device of the present invention comprises a mouthpiece that is formed in a U-shape according to a wearer's dentition and is fit in an oral cavity, and a light source that is disposed in the mouthpiece and is provided with a plurality of light emitters, such that the intraoral light therapy device irradiates light to the wearer's gums from the light emitters,
wherein the mouthpiece comprises:
   a bite tray formed in a U-shape according to the wearer's dentition and bitten by the dentition of the wearer's upper and lower jaws;
   a front flange formed vertically along an outer edge of the bite tray and facing outer surfaces of the wearer's upper and lower gums; and
   a rear flange formed vertically along an inner edge of the bite tray and facing inner surfaces of the wearer's upper and lower gums; and
wherein the front and rear flanges are optically transparent, and the light source is disposed to be capable of irradiating light from an inner peripheral surface of the front flange and/or an outer peripheral surface of the rear flange,
characterized in that each outer edge end portion of rear end portions of the bite tray is formed flush with a tip portion of a rear end portion of the front flange to which the outer edge end portion is connected, and both rear end portions of the rear flange are located anterior to the outer edge end portions of both the rear end portions of the bite tray.

In a preferred example of the intraoral light therapy device, each inner edge end portion of the rear end portions of the bite tray is formed flush with a tip portion of the rear end portion of the rear flange to which the inner edge end portion is connected so that each of the rear end portions of the bite tray is inclined forward from the outer edge end portion to the inner edge end portion in a plan view. Thus, it is possible to reduce stimulus by both the rear end portions of the bite tray to a rear portion of a tongue, such as portions of the tongue touching lower molar teeth so that a vomiting reflex can be reduced, and it is possible to stably hold the mouthpiece by biting the respective outer edge end portions of both the rear end portions of the bite tray with the molars.

More preferably, each inclined shape of both the rear end portions of the bite tray is a curved shape, such that the curvature of which changes to have a convex shape in an outer end side, a concave shape in an inner end side, and therebetween, an inverted portion between the convex and concave shapes.

In another preferred example of the intraoral light therapy device, the light source is set to irradiate light of a wavelength selected from at least within a range of 380 to 500 nm. When the light of a wavelength selected from within a range of 380 to 500 nm (so-called blue light) is irradiated onto gingival margins and free gingiva in the vicinities thereof, it is possible to obtain an effect that a vibration energy of the light can act on and sterilize harmful bacteria in the oral cavity, particularly in periodontium, thereby noninvasively improving periodontal diseases such as gingivitis and periodontitis, and preventing and treating halitosis. The wavelength of the blue light is preferably chosen from within the range of 390 to 470 nm.

More preferably, the light source is further set to irradiate light of a wavelength selected from within a range of 600 to 1,000 nm. Irradiating red light (wavelength: 600 nm to 770 nm) and/or near-infrared light (wavelength: more than 770 nm to 1,000 nm or less) can promote blood circulation in the gums and alleviate gum pain or discomfort.

The red light is particularly effective for the alleviation of pain on the outer surface of the gums, and the near-infrared light is particularly effective for the promotion of blood circulation of the gums by giving a thermal stimulus on the gums, the alleviation of pain inside the gums, a treatment for an alveolar bone, etc. The wavelength of the red light is preferably within a range of 640 nm or more to 770 nm or less. Thus, it is furthermore preferable that the light source is set to simultaneously irradiate the light of a wavelength selected from within a range of 640 nm or more to 770 nm or less and the light of a wavelength selected from within a range of more than 770 nm to 1,000 nm or less.

### ADVANTAGES OF INVENTION

Since the intraoral light therapy device of the present invention has the configuration that each outer edge end portion of the rear end portions of the bite tray is formed flush with the tip portion of the rear end portion of the front flange to which the outer edge end portion is connected, and both rear end portions of the rear flange are located anterior to the outer edge end portions of both the rear end portions of the bite tray, it is possible to reduce stimulus by the rear flange on a rear portion of a tongue or on a root of the tongue and the vicinity thereof. Thus, the intraoral light therapy device of the present invention can not only accurately irradiate light to gingival margin and free gingiva throughout a user's entire dentition, but also provide excellent wearing comfort by reducing discomfort or vomiting reflex when the user fits the mouthpiece deep inside the oral cavity.

### BRIEF DESCRIPTION OF THE DRAWINGS

Fig. 1 is a perspective view showing the intraoral light therapy device according to an example of the present invention.
Fig. 2 is a top plan view showing the intraoral light therapy device shown in Fig. 1.
Fig. 3 is a front elevational view showing the intraoral light therapy device shown in Fig. 1.
Fig. 4 is a rear elevational view showing the intraoral light therapy device shown in Fig. 1.
Fig. 5 is a side elevational view showing the intraoral light therapy device shown in Fig. 1.
Fig. 6 is a perspective view showing the intraoral light therapy device according to another example of the present invention.
Fig. 7 is a top plan view showing the intraoral light therapy device shown in Fig. 6.
Fig. 8 is a cross-sectional view showing a state where the intraoral light therapy device is fit into an oral cavity.
Fig. 9 (a) is an explanatory drawing showing positions of upper and lower recess portions of a front flange when the intraoral light therapy device is fit into the oral cavity, and Fig. 9 (b) is an explanatory drawing showing positions of upper and lower recess portions of a rear flange when the intraoral light therapy device is fit into the oral cavity.
Fig. 10 is an explanatory drawing showing positions of both rear end portions of the rear flange when the intraoral light therapy device is fit into the oral cavity.
Fig. 11 is a perspective view showing a state where the intraoral light therapy device shown in Fig. 1 is connected to a controller by an electric cable, and showing a power supply unit connected to the controller by a power source cable.
Fig. 12 is a top plan view showing a state where a power source pack with a control function is connected to the intraoral light therapy device shown in Fig. 1.
Fig. 13 is a function block diagram showing a configuration of the controller according to an example of the present invention.
Fig. 14 is a function block diagram showing a configuration of the intraoral light therapy device according to an example of the present invention.

### EMBODIMENTS OF THE INVENTION

The present invention is explained in detail by referring to an embodiment according to the drawings. However, the present invention is not restricted to the following description, and various modifications may be made within the scope of the technical thought of the present invention.

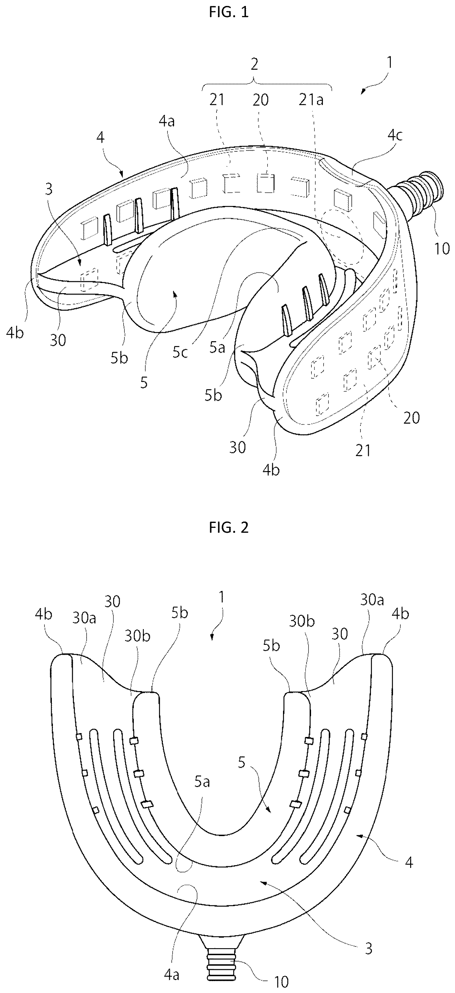

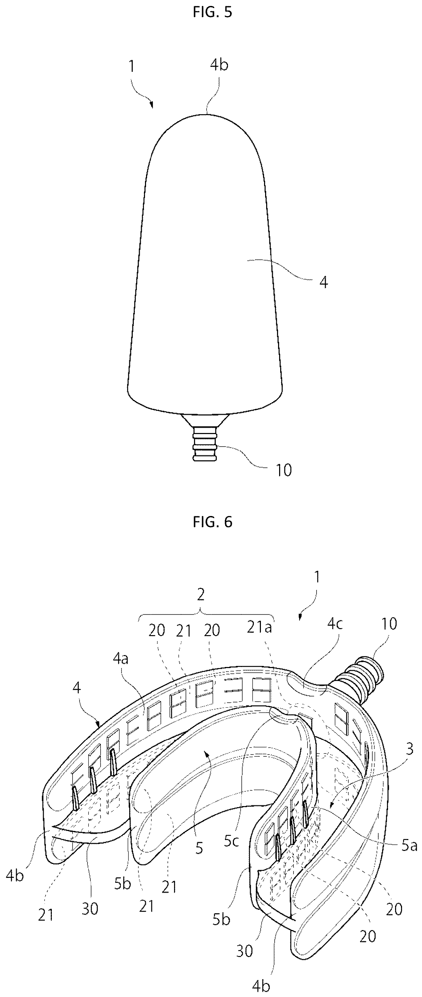

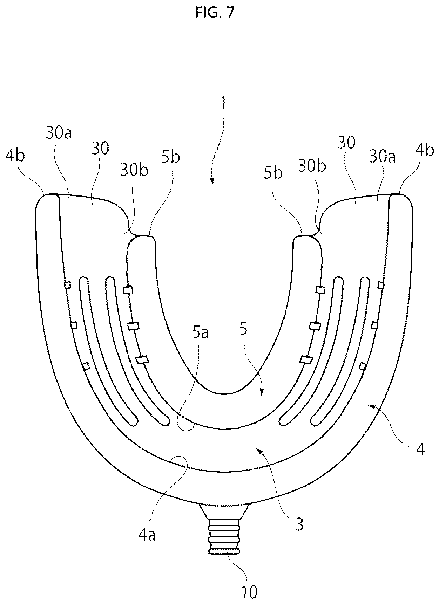

Figs. 1 to 5 show the intraoral light therapy device according to an example (the first example) of the present invention. Figs. 6, 7 show the intraoral light therapy device according to another example (the second example) of the present invention. As shown in Figs. 1 to 7, the intraoral light therapy device of the present invention includes a mouthpiece 1 formed in a U-shape according to a wearer's dentition and fit in an oral cavity, and a light source 2 disposed in the mouthpiece 1 and having a substrate 21 in which a plurality of light emitters 20 is arranged.

### [1] Mouthpiece

The mouthpiece 1 includes a bite tray 3 formed in a U-shape according to the wearer's dentition, a front flange 4 formed vertically along an outer edge of the bite tray 3 to face outer surfaces of the wearer's gums, particularly at least gingival margin thereof, and a rear flange 5 formed vertically along an inner edge of the bite tray 3 to face inner surfaces of the wearer's gums, particularly at least the gingival margin thereof. The mouthpiece 1 is used so as to cover the wearer's upper and lower dentition. The bite tray 3, the front flange 4, and the rear flange 5 maintain their respective shapes while exhibiting moderate flexibility, and are made fittable by following a shape of the inside of the oral cavity. The mouthpiece 1 has a shape that is both bilaterally symmetrical and vertically symmetrical. Incidentally, in the present specification, the outer surfaces of the gums refer to the surfaces facing the cheeks and lips, and the inner surfaces of the gums refer to the surfaces opposite the outer surfaces of the gums, facing the tongue.

The bite tray 3 is formed in a rough U-shape in a plan view according to the wearer's dentition shape, and is a part where the wearer can bite when the wearer holds the mouthpiece 1 in the oral cavity. The bite tray 3 is formed to connect a central portion in a height direction (vertical direction) of an inner peripheral surface 4a of the front flange 4 and a central portion in the height direction (vertical direction) of an outer peripheral surface 5a of the rear flange 5. Thus, when the mouthpiece 1 is fit into the oral cavity, the upper and lower dentition is inserted between the front and rear flanges 4, 5. The width of the bite tray 3 (the distance between the inner peripheral surface 4a of the front flange 4 and the outer peripheral surface 5a of the rear flange 5) may be set such that the inner peripheral surface 4a of the front flange 4 and the outer peripheral surface 5a of the rear flange 5 adhere to the gums with moderate pressure. As necessary, the width of the bite tray 3 may be set such that an appropriate gap is respectively formed between the inner peripheral surface 4a of the front flange 4 and the gums, and between the outer peripheral surface 5a of the rear flange 5 and the gums.

The front and rear flanges 4, 5, particularly the front flange 4, are formed to face at least molars (second molars) and, if necessary, to face wisdom teeth (third molars), and are designed to be capable of irradiating light from the light emitters 20 to at least the gingival margins and the vicinity thereof. A height (vertical length) of each of the front and rear flanges 4, 5 may be in a degree such that, when the mouthpiece 1 is fit into the oral cavity, the light emitters 20 disposed in the light source 2 can sufficiently emit light to the outer surfaces and inner surfaces of the wearer's gums, particularly at least the gingival margins and the vicinities thereof. Incidentally, in the first example, as shown in Fig. 1, the light source 2 is disposed in the front flange 4 only to irradiate the light to the outer surfaces of the wearer's gums only, and in the second example, as shown in Fig. 6, the light source 2 is disposed in both the front and rear flanges 4, 5 to irradiate the light to both the outer surfaces and inner surfaces of the wearer's gums. These differences in the arrangement of the light source 2 can be appropriately selected according to the purpose of treatment, the disease state, the bacteria to be eliminated, etc.

The front and rear flanges 4, 5 are formed such that, in a side view, their respective heights gradually decrease from the vicinity of the front end portion to the rear end. Thus, the mouthpiece 1 can be easily fit into and removed from the oral cavity, and stimulus on a root of the tongue and the vicinity thereof (for example, a portion of the tongue touching the lower molar teeth) by the rear flange 5 can be reduced, so that a vomiting reflex can be suppressed. A degree of this gradual decrease may be within a range that the light can be sufficiently irradiated to the outer surfaces and inner surfaces of the wearer's gums, particularly at least the gingival margins and the vicinity thereof, from the light emitters 20 disposed in both rear end portions 4b, 4b of the front flange 4 and both rear end portions 5b, 5b of the rear flange 5.

At least the inner peripheral surface 4a of the front flange 4 and the outer peripheral surface 5a of the rear flange 5 are optically transparent. The light emitters 20 are arranged in the light source 2 such that the light is irradiated from the inner peripheral surface 4a of the front flange 4 in the first example, and such that the light is irradiated from the inner peripheral surface 4a of the front flange 4 and the outer peripheral surface 5a of the rear flange 5 in the second example, respectively. Thus, it is made to be capable of irradiating the light from the light emitters 20 to the outer surfaces of the wearer's gums only (the first example) or the outer surfaces and inner surfaces of the wearer's gums (the second example), particularly it is made to be capable of irradiating the light to at least the gingival margins and the vicinity thereof. Incidentally, in the present specification, each of the outer peripheral surfaces of the front and rear flanges 4, 5 refers to the surface fronting on the outer side of the oral cavity and facing the cheeks and lips, and each of the inner peripheral surfaces of the front and rear flanges 4, 5 refers to the surface fronting on the inner side of the oral cavity, opposite the paired outer peripheral surface, and facing the tongue.

When the mouthpiece 1 is fit into the oral cavity, each of the rear end portions 4b, 4b of the front flange 4 is inserted into a deep interior of the oral cavity and is placed on the side of the cheek with respect to the molar. Thus, the mouthpiece 1 is fit into the oral cavity snugly and can be held stably with a light pressure without the need to bite down hard.

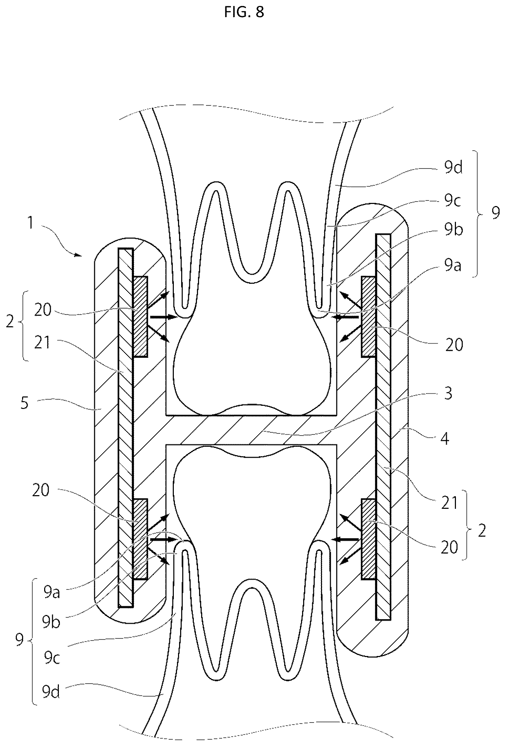

As shown in Fig. 8, when the mouthpiece 1 is fit in the oral cavity, the front flange 4 is opposite to the outer surfaces of the gums 9, particularly at least the gingival margins 9a and the vicinity thereof. The front flange 4 is optically transparent, is provided with the light source 2, and is made to be capable of irradiating the light to the outer surfaces of the gums 9, particularly to at least the gingival margins 9a and the free gingiva 9b in the vicinities thereof, from the light emitters 20 of the light source 2. As necessary, the front flange 4 may be made to be capable of irradiating the light to the attached gingiva 9c and its upper or lower alveolar mucosa 9d of the outer surfaces of the gums 9, too.

As shown in Fig. 8, when the mouthpiece 1 is fit in the oral cavity, the rear flange 5 is opposite to the inner surfaces of the gums 9, particularly at least the gingival margins 9a and the vicinity thereof. The rear flange 5 is optically transparent, is provided with the light source 2 as necessary, and is made to be capable of irradiating the light to the inner surfaces of the gums 9, particularly to at least the gingival margins 9a and the free gingiva 9b in the vicinities thereof, from the light emitters 20 of the light source 2. As necessary, the rear flange 5 may be made to be capable of irradiating the light to the attached gingiva 9c and its upper or lower alveolar mucosa 9d of the inner surfaces of the gums 9, too.

Thus, the mouthpiece 1 is configured such that tooth crowns are positioned on the upper surface and lower surface sides of the bite tray 3, the gingival margin 9a and the free gingiva 9b of the respective outer surfaces of the gums 9 are respectively positioned on the upper edge portion and lower edge portion sides of the front flange 4, and the gingival margin 9a and the free gingiva 9b of the respective inner surfaces of the gums 9 are respectively positioned on the upper edge portion and lower edge portion sides of the rear flange 5.

### (1) Recess portion

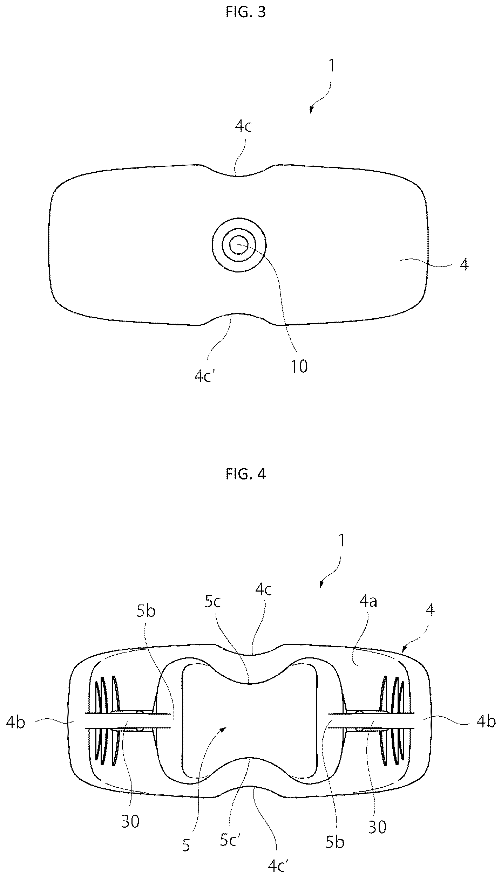

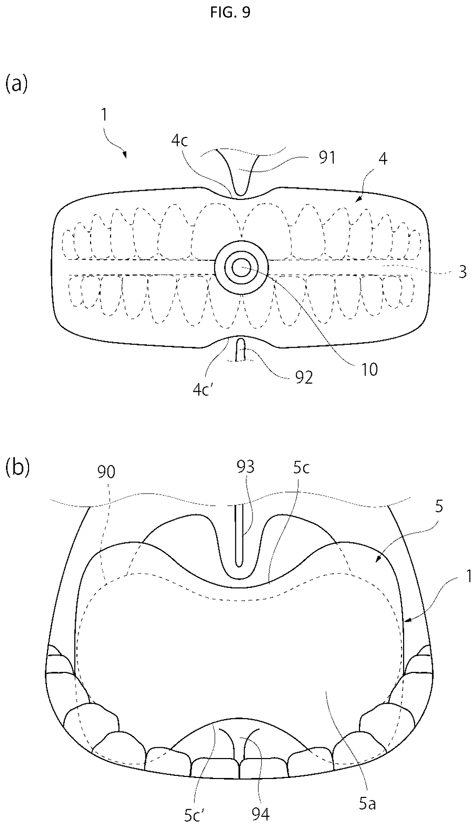

As shown in Fig. 3, the central portion of the front flange 4 facing a median line of the upper dentition is formed with an upper front recess portion 4c that is recessed in the lower side (in the side of the bite tray 3) from the upper edge portion, and the central portion of the front flange 4 facing a median line of the lower dentition is formed with a lower front recess portion 4c' that is recessed in the upper side (in the side of the bite tray 3) from the lower edge portion. As shown in Fig. 9 (a), when the mouthpiece 1 is fit into the oral cavity, an upper labial frenulum (superior labial frenulum) 91 is inserted into the upper front recess portion 4c, and a lower labial frenulum (inferior labial frenulum) 92 is inserted into the lower front recess portion 4c'. This allows the front flange 4 to fit against the upper and lower portions of the oral cavity, where the front flange 4 faces the gingival margins 9a and the free gingiva 9b, without interference between the front flange 4 and the upper and lower labial frenulums 91, 92.

On the respective backsides of the upper and lower lips, near the median line of the upper and lower dentition, the upper labial frenulum 91 and the lower labial frenulum 92 extend from the alveolar mucosa 9d to the attached gingiva 9c, respectively. Thus, when the upper and lower front recess portions 4c, 4c' are not formed on the central portion of the front flange 4 facing the median line of the upper and lower dentition, the upper edge portion of the front flange 4 interferes with the upper labial frenulum 91, or the lower edge portion of the front flange 4 interferes with the lower labial frenulum 92, and therefore it is difficult to fit the front flange 4 to the upper portion of the oral cavity, that is, the top of the backside of the upper lip, and to the lower portion of the oral cavity, that is, the bottom of the backside of the lower lip. Moreover, pain or discomfort occurs owing to the interference between the upper edge portion of the front flange 4 and the upper labial frenulum 91 or the interference between the lower edge portion of the front flange 4 and the lower labial frenulum 92 so that the height of the front flange 4 cannot be sufficiently secured, and it makes it difficult to sufficiently face the front flange 4 to the gingival margins 9a and the free gingiva 9b of the vicinity thereof. The depth of the upper front recess portion 4c may be such that it does not interfere with the upper labial frenulum 91, and the depth of the lower front recess portion 4c' may be such that it does not interfere with the lower labial frenulum 92.

Accordingly, since the front flange 4 is formed with the upper and lower front recess portions 4c, 4c', it is possible to fit the front flange 4 to the top of the backside of the upper lip and the bottom of the backside of the lower lip so as to face the front flange 4 to the gingival margins 9a and the free gingiva 9b without the interference between the upper edge portion of the front flange 4 and the upper labial frenulum 91 or the interference between the lower edge portion of the front flange 4 and the lower labial frenulum 92.

As shown in Fig. 4, the central portion of the rear flange 5 facing the median line of the upper dentition is formed with an upper rear recess portion 5c that is recessed in the lower side (in the side of the bite tray 3) from the upper edge portion, and the central portion of the rear flange 5 facing the median line of the lower dentition is formed with a rear recess portion 5c' that is recessed in the upper side (in the side of the bite tray 3) from the lower edge portion. As shown in Fig. 9 (b), when the mouthpiece 1 is fit into the oral cavity, a median palatine raphe (palatine raphe) 93 is inserted into the upper rear recess portion 5c, and a lingual frenulum (frenulum linguae) 94 is inserted into the lower rear recess portion 5c'. This allows the rear flange 5 to fit against the upper and lower portions of the oral cavity, where the rear flange 5 faces the gingival margins 9a and the free gingiva 9b, without interference between the rear flange 5 and the median palatine raphe 93 or the lingual frenulum 94.

The median palatine raphe 93 extends on the palate near the median line of the upper dentition, and the lingual frenulum 94 extends below the tongue 90 near the median line of the lower dentition. Thus, when the upper and lower rear recess portions 5c, 5c' are not formed on the central portion of the rear flange 5 facing the median line of the upper and lower dentition, the upper edge portion of the rear flange 5 interferes with the median palatine raphe 93, or the lower edge portion of the rear flange 5 interferes with the lingual frenulum 94, and therefore it is difficult to fit the rear flange 5 to the upper portion of the oral cavity, that is, the top of the palate, and to the lower portion of the oral cavity, that is, the bottom of the tongue 90. Moreover, pain or discomfort occurs owing to the interference between the upper edge portion of the rear flange 5 and the median palatine raphe 93 or the interference between the lower edge portion of the rear flange 5 and the lingual frenulum 94 so that the height of the rear flange 5 cannot be sufficiently secured, and it makes it difficult to sufficiently face the rear flange 5 to the gingival margins 9a and the free gingiva 9b of the vicinity thereof. The depth of the upper rear recess portion 5c may be such that it does not interfere with the median palatine raphe 93, and the depth of the lower rear recess portion 5c' may be such that it does not interfere with the lingual frenulum 94.

Accordingly, since the rear flange 5 is formed with the upper and lower rear recess portions 5c, 5c', it is possible to fit the rear flange 5 to the top of the palate and the bottom of the tongue 90 so as to face the rear flange 5 to the gingival margins 9a and the free gingiva 9b without the interference between the upper edge portion of the rear flange 5 and the median palatine raphe 93 or the interference between the lower edge portion of the rear flange 5 and the lingual frenulum 94.

### (2) Shape of rear end portions of mouthpiece

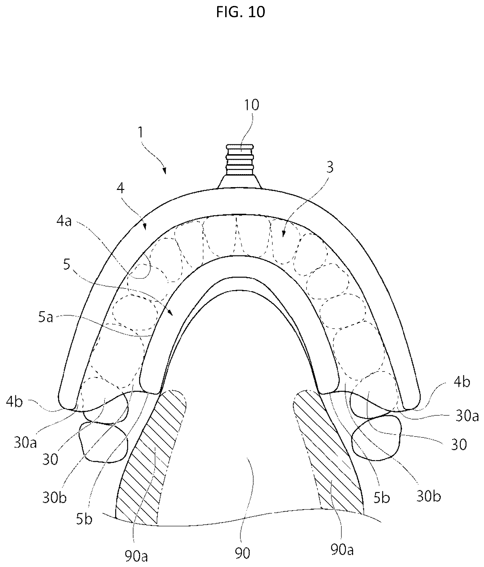

As shown in Figs. 2, 7, both the rear end portions 5b, 5b of the rear flange 5 are formed to be located anterior to both the rear end portions 4b, 4b of the front flange 4. Since both the rear end portions 5b, 5b of the rear flange 5, which are fit between the dentition and the root of the tongue side of the tongue 90, are located anterior to both the rear end portions 4b, 4b of the front flange 4, each of which is fit between the inside of the cheek and the dentition, as shown in Fig. 10, it is possible to reduce stimulus by the rear flange 5 to the rear portion of the tongue 90, the root of the tongue, and the vicinities thereof, such as portions 90a, 90a of the tongue 90 touching the lower molar teeth so that a vomiting reflex can be suppressed and a good wearing comfort can be obtained.

The positions of both the rear end portions 5b and 5b of the rear flange 5 may be set, for example, in front of the portions in the rear side (the root side) of the tongue 90 where a vomiting reflex is likely to occur by stimulus (for example, the portions 90a, 90a of the tongue 90 touching the lower molar teeth). For example, as shown in Fig. 10, both the rear end portions 5b, 5b of the rear flange 5 are positioned forward of the center of the second molars. The positions of both the rear end portions 4b, 4b of the front flange 4 may be set where the mouthpiece 1 can be fitted snugly in the oral cavity and can be held stably with a light pressure without having to bite down hard on the mouthpiece 1, behind both the rear end portions 5b, 5b of the rear flange 5. Preferably, the positions of both the rear end portions 4b, 4b of the front flange 4 are set to face at least the molars (the second molars), and as necessary, to face the wisdom teeth (the third molars).

An outer edge end portion 30a of each rear end portion 30, 30 of the bite tray 3 is formed flush with a tip portion of the rear end portion 4b of the front flange 4 to which the outer edge end portion 30a is connected, an inner edge end portion 30b of each of the rear end portions 30, 30 of the bite tray 3 is formed flush with a tip portion of the rear end portion 5b of the rear flange 5 to which the inner edge end portion 30b is connected, that is, both the rear end portions 5b, 5b of the rear flange 5 and the respective inner edge end portions 30b of the rear end portions 30, 30 of the bite tray 3 are located anterior to the respective outer edge end portions 30a of the of the rear end portion 30, 30 of the bite tray 3, and thus each of the rear end portions 30 of the bite tray 3 is inclined forward from the outer edge end portion 30a to the inner edge end portion 30b in a plan view.

Since each of the rear end portions 30 of the bite tray 3 is inclined forward from the outer edge end portion 30a to the inner edge end portion 30b, as shown in Fig. 10, it is possible to reduce stimulus by both the rear end portions 30, 30 of the bite tray 3 to the rear portion of the tongue 90, such as the portions 90a, 90a of the tongue 90 touching the lower molar teeth so that a vomiting reflex can be reduced, and it is possible to stably hold the mouthpiece 1 by biting the respective outer edge end portions 30a, 30a of both the rear end portions 30, 30 of the bite tray 3 with the molars.

The inclined shape of both the rear end portions 30, 30 of the bite tray 3 is not restricted; it can be a linear shape or a curved shape. Preferably, as shown in Figs. 2, 7, each inclined shape of both the rear end portions 30, 30 of the bite tray 3 is a curved shape such that the curvature of which changes to have a convex shape in an outer end side, a concave shape in an inner end side, and therebetween, an inverted portion between the convex and concave shapes. When the inclined shape is such a curved shape, assuming that a straight-line distance from the outer end to the inner end of the inclined shape is 100% (the sum of the straight-line distance between both ends of the convex portion and the straight-line distance between both ends of the concave portion is 100%), the ratio of the convex portion (the ratio of the straight-line distance between the two ends of the convex portion) is preferably 30 to 90%, and more preferably 40 to 90%, in terms of the straight line passing through the outer and inner ends of the inclined shape. This ratio is approximately 60% in the first example, and approximately 88% in the second example. When it is desired to reduce the stimulus to the rear portion of the tongue 90 by both the rear end portions 30, 30 of the bite tray 3 as much as possible in order to emphasize a suppression effect of a vomiting reflex, the ratio of the convex portion may be made to be relatively small, for example, 30 to 70%.

Both the rear end portions 4b, 4b of the front flange 4 and both the rear end portions 5b, 5b of the rear flange 5 may have either an arc shape as shown in Fig. 1 (the first example) or a linear shape as shown in Fig. 6 (the second example) in a side view. As in the first example, when both the rear end portions 4b, 4b of the front flange 4 and both the rear end portions 5b, 5b of the rear flange 5 are arc-shaped, fitting the mouthpiece 1 into the oral cavity and removing it from the oral cavity is relatively easy. Furthermore, since the reduction effect of the stimulus on the root of the tongue and the vicinity thereof, for example, the portions 90a, 90a of the tongue 90 touching the lower molar teeth, is relatively high, resulting in a relatively high reduction effect on the vomiting reflex. As in the second example, when both the rear end portions 4b, 4b of the front flange 4 and both the rear end portions 5b, 5b of the rear flange 5 have a linear shape, the light emitters 20 can be disposed as far as each of the rear end sides of both the rear end portions 4b, 4b and both the rear end portions 5b, 5b respectively, and the light emitters 20 can be disposed as far as each of the upper and lower edge portions of both the rear end portions 4b, 4b and both the rear end portions 5b, 5b respectively. Thus, such a configuration is advantageous when the light is to be irradiated as deep as possible to the interior of the oral cavity, and the front flange 4 can be fitted to the upper portion of the oral cavity, i.e., the top of the back side of the upper lip, and to the lower portion of the oral cavity, i.e., the bottom of the back side of the lower lip to hold the mouthpiece 1 stably.

As shown in Figs. 2 and 7, each tip surface of both the rear end portions 4b, 4b of the front flange 4 and both the rear end portions 5b, 5b of the rear flange 5 is formed in an arc shape in a plan view, and therefore, even when the rear end portions 4b, 4b and the rear end portions 5b, 5b contact the buccal mucosa respectively, a good wearing comfort is obtained.

### (3) Material

The material of the mouthpiece 1 is the same as that of a mouthpiece conventionally used in the field of sports and medicine, and is formed of a transparent or translucent resin material capable of irradiating light from the light source 2 and having flexibility. Specifically, the material for the mouthpiece 1 can be a resin that conforms to the shape of the oral cavity and has appropriate hardness and softness, such as olefin-based resins, polyester-based resins, urethane-based resins like polyurethane, polyimide-based resins, silicone-based resins, styrene-based resins, acrylic-based resins, polyamide-based resins, and carbonate-based resins. However, it is not limited to these materials.

As the above-described olefin-based resins, the following are preferably exemplified: polyethylene (PE), polyethylene-based resins, polypropylene (PP), polypropylene-based resins, ethylene-vinyl acetate copolymer (EVA), etc., and the following are more preferably exemplified: polyethylene (PE), polyethylene-based resins, polypropylene (PP), polypropylene-based resins, etc.

Polyester-based resins are polycondensates of polycarboxylic acids (such as dicarboxylic acids) and polyalcohol (such as diols). As the above-described polyester-based resins, polyethylene terephthalate (PET) is exemplified.

Urethane-based resins are polycondensates formed from a compound containing isocyanate groups and a compound containing hydroxyl groups. As the above-described urethane-based resins, thermoplastic polyurethane (TPU) is exemplified.

Polyamide-based resins are (co)polymers formed by the bonding of numerous monomers via amide bonds. As the above-described polyamide-based resins, there are examples, such as nylon, para-type amides, and meta-type amides.

Acrylic rubber-based resins are (co)polymers containing acrylic-based rubbers as main components. As the above-described acrylic-based resins, there are examples, such as a block copolymer of methyl methacrylate and butyl acrylate.

Incidentally, the bite tray 3 and the front and rear flanges 4, 5 may be formed from different materials to vary their hardness.

The above-described mouthpiece 1 can be obtained by molding the above-described material integrally with the light source 2. An injection condition for the material is set according to the size and shape of the mouthpiece 1, and typically, the entire mouthpiece 1 is molded in a single mold. The size and shape of the mouthpiece 1 may be suitable for conforming to the dentition of an average person. The mouthpiece 1 of the present invention is manufactured as an adult mouthpiece with a size and shape corresponding to the dentition of an average adult when the wearer is an adult, and as a child mouthpiece with a size and shape corresponding to the dentition of an average child when the wearer is a child.

### (4) Light source

The light source 2 includes the substrate 21 and the plurality of light emitters 20 arranged on the substrate 21. The intraoral light therapy device kills harmful bacteria present in the oral cavity, particularly within the periodontium, by irradiating blue light from the light emitters 20 onto at least the gingival margins 9a and the free gingiva 9b in the vicinities thereof, thereby noninvasively improving periodontal diseases such as gingivitis and periodontitis, and preventing and treating halitosis. Furthermore, the light emitters 20 can promote blood circulation in the gums and alleviate gum pain or discomfort by irradiating them with red light and/or near-infrared light.

### (4-1) Substrate

The substrates 21 are respectively disposed along the side surfaces of the front and rear flanges 4, 5 of the mouthpiece 1. Respective of them have an approximately rectangular plate shape corresponding to the shape of the front and rear flanges 4, 5, and are bent into a U-shape to be disposed. In the first example, one piece of the substrate 21, which has an approximately rectangular plate shape according to the shape of the front flange 4, is bent into a U-shape along the side surface over almost the entire area of the front flange 4 to be disposed. In the second example, two pieces of the substrate 21, each of which has an approximately rectangular plate shape according to the shape of the front flange 4, are respectively bent into a U-shape along the upper and lower edge portions of the front flange 4 to be disposed, and two pieces of the substrate 21, each of which has an approximately rectangular plate shape according to the shape of the rear flange 5, are respectively bent into a U-shape along the upper and lower edge portions of the rear flange 5 to be disposed. However, it is not restricted to these configurations.

The light emitters 20 are arranged on the substrate 21 such that the light is emitted only from the inner peripheral surface of the front flange 4 (the first example), or from both the inner peripheral surface of the front flange 4 and the outer peripheral surface of the rear flange 5 (the second example). Thus, in the first example, the light source 2 faces only the outer surfaces of the wearer's gums 9, enabling the light to be irradiated onto the gingival margins 9a of the outer surfaces of the gums 9 and the free gingiva 9b in the vicinities thereof, and in the second example, the light sources 2 are positioned to face each other between the front and rear flanges 4, 5, enabling light to be irradiated onto the gingival margins 9a of the outer and inner surfaces of the gums 9 and the free gingivas 9b in the vicinities thereof.

As shown in Figs. 1, 6, the substrate 21 disposed in the front flange 4 has a connecting terminal part 21a formed in the central portion facing the median line of the upper and lower dentition, and as shown in Figs. 1, 3, 6, 11, a lead-out part 10 is provided on the central portion of the outside surface of the front flange 4, through which an electric cable 60 is led out. Each of the light emitters 20 on the substrate 21 disposed in the front flange 4 is connected to the connecting terminal part 21a via an internal wiring. Each of the light emitters 20 on the substrate 21 disposed in the rear flange 5 is connected to the connecting terminal part 21a via another internal wiring passing through the bite tray 3 at the central portion facing the median line of the upper and lower dentition.

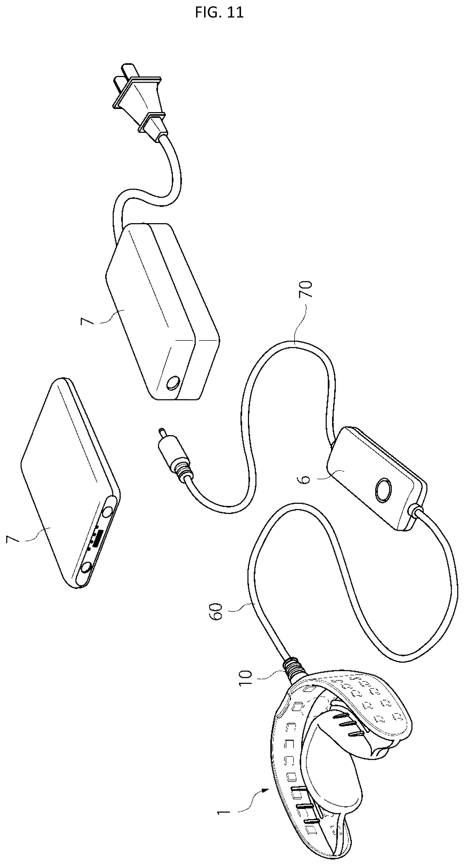

As shown in Fig. 11, the connecting terminal part 21a is connected to a controller 6 via an electric cable 60 led out from the outside surface of the central portion of the front flange 4 of the mouthpiece 1. The light source 2 is controlled by the controller 6 connected to the end portion of the electric cable 60 to regulate light irradiation by the light emitters 20 (irradiation ON and OFF, irradiation area, irradiation mode (duration, radiation intensity, irradiation dose, etc.)). The controller 6 is further detachably connected to a power supply unit 7 via a power cable 70.

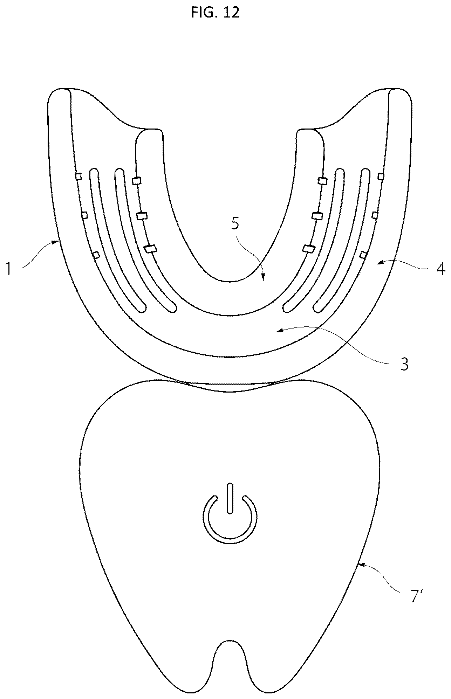

As shown in Fig. 12, the connecting terminal part 21a may also be detachably connected to a power source pack 7' with a control function that incorporates a controller capable of controlling the light source 2, without a cable.

As the substrate 21, there are no particular restrictions as long as it is a flexible substrate having flexibility and capable of mounting the light emitters 20. For example, a known flexible substrate using polyimide (PI), liquid crystal polymer (LCP), or the like as a base film can be used.

### (4-2) Light emitters

The light emitters 20 are not particularly restricted. As the light emitters 20, there are examples, such as light-emitting diode (LED), organic light-emitting diode (OLED), semiconductor laser diode (LD), polymer light-emitting diode (PLED), light-emitting polymer (LEP), optical fiber bundle, etc., or combinations thereof, but are not limited to these. The light emitters 20 are covered with transparent or translucent resin constituting the mouthpiece 1 by forming the light source 2 integrally with the mouthpiece 1. Thus, the light source 2 is made to be capable of irradiating the light in the oral cavity, and is protected by the resin material when the mouthpiece 1 is used or cleaned.

The plurality of light emitters 20 is mounted along the longitudinal direction of the substrate 21, extending from one end to the other. In the first example, on the substrate 21 of the front flange 4, the light emitters 20 are arranged in 2 columns of 14 to 16 pieces to irradiate the upper and lower dentition, respectively. In the second example, on each of the upper and lower substrates 21 of the front flange 4, the light emitters 20 are arranged in 2 columns of 18 to 20 pieces, and on each of the upper and lower substrates 21 of the rear flange 5, the light emitters 20 are arranged in 2 columns of 10 to 12 pieces. Incidentally, the arrangement pattern and number of the light emitters 20 are not limited to these examples. For example, although the light source 2 is disposed in the front flange 4 only in the first example, the rear flange 5 may also be disposed with the light source 2, in which the light emitters 20 are arranged in 2 columns to irradiate the upper and lower dentition respectively.

The light emitters 20 constituting the light source 2 include first light emitters emitting blue light (wavelength: 380 to 500 nm), second light emitters emitting red light (wavelength: 600 nm to 770 nm), and third light emitters emitting near-infrared light (wavelength: more than 770 nm to 1,000 nm or less). Incidentally, in the present specification, it is sometimes referred to as "the light emitters 20" when the first to third light emitters are not distinguished or when all the first to third light emitters are referred to.

As the first light emitters, there are examples, such as semiconductor laser diode (LD), light-emitting diode (LED), and organic light-emitting diode (OLED), each of which emits blue light (wavelength: 380 to 500 nm). As the second light emitters, there are examples, such as LD, LED, and OLED, each of which emits red light (wavelength: 600 nm to 770 nm). As the third light emitters, there are examples, such as LD, LED, and OLED, each of which emits near-infrared light (wavelength: more than 770 nm to 1,000 nm or less).

When the blue light is irradiated, the vibration energy of the light can act on and sterilize harmful bacteria in the oral cavity, particularly in the periodontium, thereby noninvasively improving periodontal diseases such as gingivitis and periodontitis, and preventing and treating halitosis. The wavelength of the blue light may be chosen appropriately based on the treatment purpose, symptom, bacteria to be killed, etc., but it is preferable to choose from within the range of 390 to 470 nm. For example, the light of a wavelength of 405 nm, 455 nm, and the like is used.

The red light is particularly effective for the alleviation of pain on the outer surface of the gums, and the near-infrared light is particularly effective for the promotion of blood circulation of the gums by giving a thermal stimulus on the gums, the alleviation of pain inside the gums, the treatment for an alveolar bone, etc. The wavelength of the red light is preferably within a range of 640 nm or more to 770 nm or less. It is preferable to irradiate the red light and near-infrared light simultaneously, for example, the red light of a wavelength of 660 nm and the near-infrared light of a wavelength of 810 nm are irradiated simultaneously.

An arrangement of the first to third light emitters on the light source 2 can be properly set according to the treatment purpose, symptom, bacteria to be killed, etc. For example, in each of the columns of the light emitters 20 in the front flange 4 of the first example shown in Fig. 1, the first light emitter can be arranged alternately, and the second light emitter and the third light emitter are arranged in order between them. Moreover, regarding each of the columns of the light emitters 20 on the sides of the most upper and the most lower edge portions in each of the front and rear flanges 4, 5 in the second example shown in Fig. 6, the second light emitter and the third light emitter are alternately arranged, and the light emitters 20 of the 2 columns inside these columns, that is, 2 columns above and below the bite tray 3 to be extended closely along the bite tray 3, can be configured as the first light emitters.

In addition, the light source 2 may include units each of which consists of a combination of the first to third light emitters, and a plurality of units is provided on the surface of the substrate 21. There are no particular limitations on the configuration of the unit, and it can be designed arbitrarily. For example, a unit having one first to third light emitters, a unit having two first light emitters, one second light emitter, and one third light emitter, and so forth, are exemplified, but are not limited to these.

The intraoral light therapy device has at least two modes for irradiating the light emitters 20. That is, the intraoral light therapy device at least has the first mode that makes only the first light emitters emit, and the second mode that makes the second and third light emitters emit simultaneously. However, the intraoral light therapy device may have a mode that makes the first to third light emitters emit simultaneously, another mode that makes the first and second light emitters emit simultaneously, another mode that makes the first and third light emitters emit simultaneously, or another mode that makes each of the first to third light emitters emit separately, etc. Switching between each of the modes is controlled by a control part 6a provided in the controller 6.

The wavelength (nm) and radiant flux (W) of the irradiation light emitted from each of the first to third light emitters are different between these emitters. Therefore, by switching the modes using the control part 6a, the intraoral light therapy device can differentiate a radiation intensity (Irradiance: W/m²) and an irradiation energy (J) for an irradiated area between the first and second modes, thereby irradiating an optimal light depending on the irradiated area and symptoms thereon.

In addition, the intraoral light therapy device can appropriately set a pattern of light irradiation in each of the modes. In the first mode, the first light emitters may be uniformly lit at the same radiation intensity or flashed. In the second mode, the second light emitters and/or the third light emitters may be uniformly lit at the same radiation intensity or flashed. When flashing them, for example, light pulses are generated. Moreover, an irradiation position, the radiation intensity, and an irradiation pattern may be set to vary with time.

For the light source to irradiate light, it may be possible to select the mode to irradiate only from the light source 2 of the front flange 4, the mode to irradiate only from the light source 2 of the rear flange 5, and the mode to irradiate from the light sources 2 of the front and rear flanges 4 and 5 by settings. Further, the irradiation area of each of the light sources of the front and rear flanges 4, 5 may be selected as the whole of the light emitters 20, half of the front teeth side, the other half of the molar side, and so forth. Moreover, the irradiation position, the radiation intensity, and the irradiation pattern may be set to vary with time.

The irradiation time for each of the first and second modes can also be set as appropriate. For example, an irradiation pattern includes a total irradiation time of 600 seconds ± 10%, where an irradiating time is set as 300 seconds ± 10% in the first and second modes, respectively.

### (5) Thermistor

Incidentally, the substrate 21 may include a temperature detection part 21b, such as a thermistor, for detecting the temperature of the light source 2. This allows the light emitters 20 to be controlled to stop the light irradiation when they are heated to a predetermined temperature or higher, thereby protecting the wearer and the mouthpiece 1.

### [2] Controller

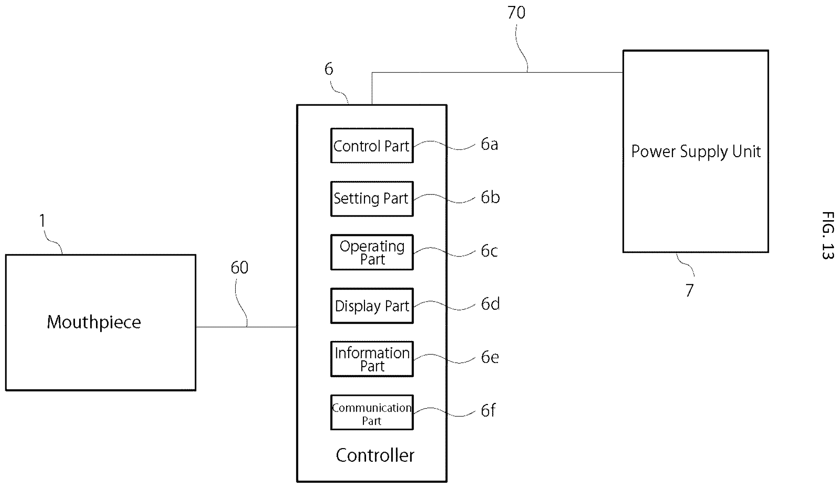

As shown in Fig. 11, the mouthpiece 1 is detachably connected to the controller 6 via the electric cable 60 led out from the outside surface of the central portion of the front flange 4, for example. As shown in Fig. 13, the controller 6 has the control part 6a and an operating part 6c, wherein the control part 6a controls the irradiation area, irradiation time, irradiation wavelength, radiation intensity, etc. of the light emitters 20 based on a program, and stores a mode in which the irradiation area, irradiation time, wavelength of irradiation light, radiation intensity, radiation energy, etc. are set in advance, and an operation history of the intraoral light therapy device in memory and storage, and wherein the operating part 6c is equipped with a control button for operation such as turning a power source ON/OFF, turning irradiation ON/OFF, and selecting modes.

The controller 6 may be provided with a setting part 6b for manually setting the irradiation area, irradiation time, irradiation wavelength, radiation intensity, irradiation energy, etc., a display part 6d, such as an LCD panel, that displays the operation condition, operation history, schedule management, remaining light irradiation time, battery level, etc. of the intraoral light therapy device, an information part 6e that notifies of the completion of a predetermined light irradiation, error states, and so forth, via alarms, light emission, vibration, etc., and a communication part 6f that connects to an external server 8 via the internet and performs renewal of a control program and a schedule stored in the control part 6a and a transmission of the operation history saved in the control part 6a.

The controller 6 is further detachably connected to the power supply unit 7 via the power cable 70. The power supply unit 7 can be a power source device (adapter) connected to a household outlet or a mobile battery containing a rechargeable or disposable battery. In this way, by separating the mouthpiece 1 from the controller 6 and the power supply unit 7, and by connecting them with cables, the weight required when fitting the mouthpiece 1 is only the weight of the mouthpiece 1 itself, and misalignment of a fitting position can be prevented, the wearer's burden of holding the mouthpiece 1 in the oral cavity can be reduced, and further improving operability. Furthermore, since the mouthpiece 1 is detachable from the power cable 70, it is easy to clean only the mouthpiece 1 after use.

As shown in Fig. 12, the power source pack 7' with a control function that incorporates a controller capable of controlling the light source 2 as described above, and stores a rechargeable secondary battery or a disposable battery, may be detachably connected to the mouthpiece 1, without a cable. The secondary battery built in the power source pack 7' with a control function may be taken out of the power source pack 7' with a control function at the time of recharging and recharged from a household outlet or the like using an external charger, or recharged by connecting a terminal provided on the power source pack 7' with a control function to a charger connected to a household outlet or the like using a cable. By using such a power source pack 7' with a control function, it is easy to carry the mouthpiece 1 and the set of controller and power source.

Incidentally, the secondary battery may be built into the controller 6 or the mouthpiece 1. The secondary battery built into the controller 6 or the mouthpiece 1 can be charged when the controller 6 or the mouthpiece 1 is connected to the power source device connected to the household outlet, via the power source cable. Additionally, the secondary battery built into the mouthpiece 1 may be rechargeable when the mouthpiece 1 is stored in a mouthpiece case (not shown). In this case, the mouthpiece case is connected to the power source device (adapter) connected to the household outlet, via the power source cable, or the mouthpiece case incorporates the power source device and is connected to the household outlet via the power source cable.

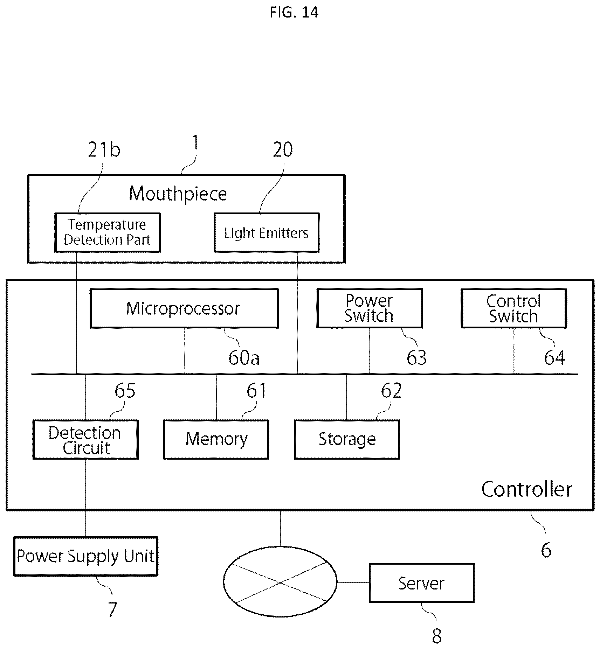

Fig. 14 is a function block diagram showing a configuration example of the intraoral light therapy device according to an embodiment of the present invention. The control part 6a includes a microprocessor 60a. The microprocessor 60a communicates with a memory 61 or a storage 62 about a program such as the irradiation mode, the operation history of the intraoral light therapy device, and so forth. The microprocessor 60a also saves the setting information of the setting part 6b in the memory 61 or the storage 62, and controls the irradiation according to the setting saved in the memory 61 or the storage 62.

The microprocessor 60a operates according to the operations of a power switch 63, which turns the power source ON/OFF, and a control switch 64, which operates the start/stop of irradiation. The microprocessor 60a also stops the light irradiation in response to a signal from the temperature detection part 21b, such as a thermistor. Thus, when the light emitters 20 are heated to a predetermined temperature or more, it is possible to control the intraoral light therapy device so that the light irradiation is stopped, so as to protect the wearer and the mouthpiece 1.

Furthermore, the microprocessor 60a receives the output of a detection circuit 65 for detecting an output voltage when the power supply unit 7 is configured with a mobile battery, judges a charge amount, and controls the display part 6d and the information part 6e to inform a user.

### [3] Usage

The usage of the intraoral light therapy device is described below. The intraoral light therapy device is used by a user for a predetermined period, for example, once to several times a day, for a predetermined time per 1 time or per day (for example, about 10 minutes per day).

### (1) Fitting

When the intraoral light therapy device is used, the mouthpiece 1 is fit to the upper and lower dentition while facing the upper and lower U-shaped grooves formed by the front flange 4, the rear flange 5, and the bite tray 3 toward the upper and lower dentition. At this time, as shown in Figs. 3 and 9 (a), since the upper and lower front recess portions 4c and 4c' are provided on the front flange 4, the mouthpiece 1 can be fit to the top of the back side of the upper lip and the bottom of the back side of the lower lip, without the interference between the front flange 4 and the upper and lower labial frenulums 91, 92. As shown in Figs. 4, 9 (b), since the upper and lower rear recess portions 5c, 5c' are provided on the rear flange 5, the mouthpiece 1 can be fit to the top of the palate and the bottom of the tongue 90, without the interference of the palatal raphe 93 and the lingual frenulum 94 with the rear flange 5. Thus, as shown in Fig. 8, the respective light sources 2 provided in the front and rear flanges 4, 5 can be made to face the gingival margins 9a and the free gingiva 9b in the vicinities thereof.

Fig. 8 is a cross-sectional view showing the positional relation between the light source 2 and the gums 9 when the intraoral light therapy device is fit into the upper and lower dentition. When the intraoral light therapy device is fit along the upper and lower dentition, the light emitters 20 are made to face the gingival margins 9a and the free gingiva 9b in the vicinities thereof to irradiate the gingival margins 9a and the free gingiva 9b with a predetermined light. As necessary, the front and rear flanges 4, 5 may be configured to be capable of irradiating light to the attached gingivas 9c and to the alveolar mucosa 9d above or below thereof.

### (2) Irradiation

As the light, a light having a wavelength selected from the wavelength range of at least so-called blue light (light having a wavelength in the range of 380 to 500 nm in general), that is, a light having a wavelength selected from the wavelength range of at least 380 to 500 nm is irradiated. When the blue light is irradiated, the vibration energy of the light can act on and sterilize harmful bacteria in the oral cavity, particularly in the periodontium, thereby noninvasively improving periodontal diseases such as gingivitis and periodontitis, and preventing and treating halitosis. The wavelength of blue light may be chosen appropriately based on the treatment purpose, symptom, bacteria to be killed, etc., but it is preferable to choose from within the range of 390 to 470 nm. For example, the light of a wavelength of 405 nm, 455 nm, and the like is used.

As the light, in addition to the above blue light, it is preferable to irradiate light of a wavelength selected from within the range of 600 to 1,000 nm, it is more preferable to irradiate red light of a wavelength selected from within the range of 600 nm or more to 770 nm or less and/or near-infrared light of a wavelength selected from within the range of more than 770 nm to 1,000 nm or less, and it is further more preferable to simultaneously irradiate both red light of a wavelength selected from within the range of 600 nm or more to 770 nm or less and near-infrared light of a wavelength selected from within the range of more than 770 nm to 1,000 nm or less. The red light of a wavelength selected from within the range of 600 nm to 770 nm is effective for the alleviation of pain on the outer surface of the gums, and the near-infrared light of a wavelength selected from within the range of more than 770 nm to 1,000 nm or less is effective for the promotion of blood circulation of the gums by giving a thermal stimulus on the gums, the alleviation of pain inside the gums, the treatment for an alveolar bone, etc. The wavelength of the red light is preferably within a range of 640 nm or more to 770 nm or less. When the above-described simultaneous irradiation is performed, for example, the red light of a wavelength of 660 nm and the near-infrared light of a wavelength of 810 nm are irradiated simultaneously.

The radiation intensity of light (Irradiance: W/m²), the irradiation area, and the irradiation time are not specifically limited. These parameters may be appropriately set to enable the light irradiated on the irradiation area, particularly the gingival margins 9a, the free gingiva 9b, and the gingival sulcus to kill harmful bacteria within the oral cavity, such as those responsible for periodontal diseases such as gingivitis and periodontitis, while simultaneously preventing damage to teeth, gums, periodontal ligament, alveolar bone, etc.

The following describes the setting examples for wavelength, radiation intensity, irradiation time, and irradiation energy quantity for the light emitters 20 in each mode of light irradiation. As described above, the surface of the substrate 21 includes: for example, the first light emitters: LEDs emitting blue light (emission wavelength: 380 to 500 nm), such as at a wavelength of 405 nm, 455 nm, and so forth; the second light emitters: LEDs emitting red light (emission wavelength: 600 to 770 nm), such as at a wavelength of 660 nm; and the third light emitters: LEDs emitting near-infrared light (emission wavelength: more than 770 nm to 1,000 nm or less), such as at a wavelength of 810 nm. Incidentally, in either mode, the operation stopping temperature of the light emitters 20 monitored by the temperature detection part 21b is set to, for example, 42°C.

In the first mode, the intraoral light therapy device is configured such that the first light emitters emit only the blue light. For example, light pulses are generated at a frequency of 10 Hz and a duty cycle of 50% such that an average optical output of each of the first light emitters is 1 W, the intensity of each of the first light emitters with respect to a target illuminance area of 0.6 cm² is set to 650 mW/cm² ± 10%, and irradiation is performed for a period of 300 sec ± 10%, thereby making an irradiation energy quantity (fluence) set approximately 200 J/cm².

In the second mode, the intraoral light therapy device is configured such that the second and third light emitters emit the red light and the near-infrared light simultaneously. For example, light pulses are generated at a frequency of 10 Hz and a duty cycle of 50% such that an average optical output of each of the second and third light emitters is 1 W, and irradiation is performed for a period of 300 sec ± 10%, thereby making a total irradiation energy quantity by the second and third light emitters set approximately 110 J/cm².

Incidentally, radiation intensity can be obtained by irradiating from the light emitters 20 and measuring the radiant flux of light incident per unit area with a radiation intensity meter. The irradiation energy quantity (J/cm²) of the light irradiated from the light emitters 20 can be calculated by multiplying the total radiation intensity (W/cm²) and the irradiation time (sec). The total radiation intensity of the intraoral light therapy device can be calculated as the sum of each radiation intensity of the light emitters 20. The selection of each mode can be performed using the controller 6.

The values of emission wavelength, radiation intensity, operating time, and irradiation energy quantity of the light emitters 20 for each mode described above are examples, and the setting values for each mode are not limited to those described above. For the intraoral light therapy device, the emission color of an indicator in the information part 6e of the controller 6 may be changed for each mode, so that it can be identified in which mode it is driven.

Using controller 6, when the operation to start irradiation is performed after selecting the mode, light irradiation is executed according to the selected mode. After the light irradiation is completed, the mouthpiece 1 can be removed from the oral cavity and cleaned.

As necessary, the light may be irradiated after applying a photosensitizer to the gums and tooth surfaces. The use of a photosensitizer in combination with the light irradiation can further enhance the bactericidal effect.

The photosensitizer can be either a naturally occurring compound (natural photosensitizer) or a synthetic compound (synthetic photosensitizer). As the natural photosensitizer, the following are exemplified: hypericin, curcumin, phenalenone derivatives, selcosporin, psoralen, xanthotoxin, angelicin, α-terthienyl, phenylthepatriyne, cannabidiol, etc. As the synthetic photosensitizer, the following are exemplified: rose bengal, methylene blue, porphyrin derivatives, curcumin derivatives, methylene blue, indocyanine green, erythrosine, phenalenone derivatives, fullerene derivatives, xanthene derivatives, resveratrol, etc.

The photosensitizer can be a mixture of the natural photosensitizer and the synthetic photosensitizer, such as a mixture of curcumin and curcumin derivatives (curcumin mixture). As other photosensitizers, extract of berries such as a lingonberry, a blueberry, and the like that contain a polyphenolic compound and/or an anthocyanin compound is exemplified.

The photosensitizer can be applied to the gums and tooth surfaces in the form of an aqueous solution, alcohol-containing solution, hydrophilic gel, hydrophobic gel, hydrophilic polymer, hydrophobic polymer, paste, lotion, or the like.

### [4] Medical effects

The following medical effects can be obtained by using the intraoral light therapy device of the present invention.
- Preventive, improvement, or curative effect for any class of periodontal disease
- Preventive, improvement, or curative effect for any class of peri-implantitis
- Preventive, improvement, or curative effect for any condition of gingivitis
- Effect in reducing infections associated with all types of periodontal surgery
- Effect in reducing pain associated with all types of periodontal surgery
- Effect in reducing bleeding in all types of periodontal disease
- Effect in reducing inflammation in all types of periodontal disease
- Effect in reducing pocket depth in all types of periodontal disease
- Effect in reducing levels of activated matrix metalloproteinase-8 (aMMP-8) in all types of periodontal diseases
- Effect in reducing microbial pathogens in all types of periodontal disease
- Effect in reducing visible plaques in all types of periodontal disease
- Effect in reducing gingival inflammation in all types of periodontal disease
- Effect in reducing the number of bacteria such as Aggregatibacter actinomycetemcomitans, Porphyromonas gingivalis, Treponema denticola, Tannerella forsythia, Prevotella intermedia, Parvimonas micra, etc. in all types of periodontal disease

### REFERENCE SIGNS LIST

- 1...: mouthpiece
10... lead-out part
- 2...: light source
20... light emitter
21... substrate
21a... connecting terminal part
21b... temperature detection part
- 3...: bite tray
30... rear end portion
30a... outer edge end portion
30b... inner edge end portion
- 4...: front flange
4a... inner peripheral surface
4b... rear end portion
4c, 4c'... front recess portion
- 5...: rear flange
5a... outer peripheral surface
5b... rear end portion
5c, 5c'.. rear recess portion
- 6...: controller
6a... control part
60a... microprocessor
6b... setting part
6c... operating part
6d... display part
6e... information part
6f... communication part
61... memory
62... storage
63... power switch
64... control switch
65... detection circuit
- 7...: power supply unit
- 7'...: power source pack with a control function
- 8...: external server
- 9...: gums
9a... gingival margin
9b... free gingiva
9c... the attached gingiva
9d... alveolar mucosa
- 60...: electric cable
- 70...: power source cable
- 90...: tongue
90a... portion touching lower molar teeth
- 91...: upper labial frenulum
- 92...: lower labial frenulum
- 93...: median palatine raphe
- 94...: lingual frenulum

## Claims

1. An intraoral light therapy device comprising a mouthpiece (1) that is formed in a U-shape according to a wearer's dentition and is fit in an oral cavity, and a light source (2) that is disposed in the mouthpiece (1) and is provided with a plurality of light emitters (20), such that the intraoral light therapy device irradiates light to the wearer's gums (9) from the light emitters (20),
wherein the mouthpiece (1) comprises:
a bite tray (3) formed in a U-shape according to the wearer's dentition and bitten by the dentition of the wearer's upper and lower jaws;
a front flange (4) formed vertically along an outer edge of the bite tray (3) and facing outer surfaces of the wearer's upper and lower gums (9); and
a rear flange (5) formed vertically along an inner edge of the bite tray (3) and facing inner surfaces of the wearer's upper and lower gums (9); and
wherein the front and rear flanges (4, 5) are optically transparent, and the light source (2) is disposed to be capable of irradiating light from an inner peripheral surface (4a) of the front flange (4) and/or an outer peripheral surface (5a) of the rear flange (5),
**characterized in that** each outer edge end portion (30a) of rear end portions (30, 30) of the bite tray (3) is formed flush with a tip portion of a rear end portion (4b) of the front flange (4) to which the outer edge end portion (30a) is connected, and both rear end portions (5b, 5b) of the rear flange (5) are located anterior to the outer edge end portions (30a, 30a) of both the rear end portions (30, 30) of the bite tray (3).

2. The intraoral light therapy device according to claim 1, wherein each inner edge end portion (30b) of the rear end portions (30, 30) of the bite tray (3) is formed flush with a tip portion of the rear end portion (5b) of the rear flange (5) to which the inner edge end portion (30b) is connected so that each of the rear end portions (30, 30) of the bite tray (3) is inclined forward from the outer edge end portion (30a) to the inner edge end portion (30b) in a plan view.

3. The intraoral light therapy device according to claim 2, wherein each inclined shape of both the rear end portions (30, 30) of the bite tray (3) is a curved shape, such that the curvature of which changes to have a convex shape in an outer end side, a concave shape in an inner end side, and therebetween, an inverted portion between the convex and concave shapes.

4. The intraoral light therapy device according to any one of claims 1 to 3, wherein the light source (2) is set to irradiate light of a wavelength selected from at least within a range of 380 to 500 nm.

5. The intraoral light therapy device according to claim 4, wherein the light source (2) is further set to irradiate light of a wavelength selected from within a range of 600 to 1,000 nm.

6. The intraoral light therapy device according to claim 5, wherein the light source (2) is set to simultaneously irradiate light of a wavelength selected from within a range of 640 nm or more to 770 nm or less and light of a wavelength selected from within a range of more than 770 nm to 1,000 nm or less.
